# EUROPEAN PATENT APPLICATION

(11) **EP 2 963 119 A1**
(43) Date of publication of application: **06.01.2016**
(21) Application number: 14756513.9
(22) Date of filing: 27.02.2014
(51) Int. Cl.: C12P 7/62, C12N 1/21, C12N 15/09

(54) **PRODUCTION METHOD FOR COPOLYMER POLYHYDROXYALKANOATE USING GENETICALLY MODIFIED STRAIN OF FATTY ACID -OXIDATION PATHWAY**

(30) Priority: 28.02.2013 JP 2013039460
(71) Applicant: Tokyo Institute of Technology, Tokyo 152-8550 (JP); Kaneka Corporation, Osaka-shi, Osaka 530-8288 (JP)
(72) Inventor: FUKUI, Toshiaki, Tokyo 152-8550 (JP); ORITA, Izumi, Tokyo 152-8550 (JP)
(74) Representative: Awapatent AB
(86) International application number: PCT/JP2014/054905
(87) International publication number: WO 2014/133088

(57) **Abstract**

An object of the present invention is to provide a method for producing poly(3-hydroxybutyrate-co-3-hydroxyhexanoate (P(3HB-co-3HHx)) having a high 3-hydroxyhexanoate fraction using a vegetable oil as a basic raw material. According to the present invention, a method is provided for producing P(3HB-co-3HHx) having a high 3-HHx fraction using a vegetable oil as a basic raw material by disrupting and so forth at least one gene encoding 2-enoyl-CoA hydratase or at least one gene encoding 3-hydroxyacyl-CoA dehydrogenase on a chromosome of a recombinant *Cupriavidus necator* strain imparted with the ability to produce P(3HB-co-3HHx).

SEQUENCE LISTING

<110>  Tokyo Institute of Technology
<120>  A process for producting polyhydroxyalkanoic acid
<130>  AB833-PCT

<150>  JP2013-039460
<151>  2013-02-28

<160>  15

<170>  PatentIn version 3.5

<210>  1
<211>  2424
<212>  DNA
<213>  Ralstonia eutropha (H16_A0461)

<400>  1
atgtccaatt tcatcgtcaa gaaggtcgcc gtgctgggtg ccggcgtcat gggggcgcag      60
atcgccgccc acctgatcaa tgcgcgcgtg ccggtggtgc tgttcgacct tcccgccaag     120
gaaggcccca agaacggcat cgcgctgcgc gccatcgaga acctgaagaa gctctcgccc     180
gcgccgctgg gcatcaagga agaggccggc ctgatccagg cggccaacta cgaggacgac     240
atcgcgctgc tcaaggaatg cgacctggtg atcgaggcga tcgccgagcg catggactgg     300
aagcacgacc tgtacaagaa ggtcgcgccg cacctggcct cgcacgcgat cttcgcgacc     360
aacacctcgg gcctgtcgat cacagcgctg tccgacgggt ttgatgcgga cctgaagtcg     420
cgcttctgcg gcgtgcactt cttcaacccg ccgcgctaca tgcacctggt ggaactgatc     480
ccgaccgcga ccacgcagcc gcagatcctc gaccagctgg aagccttcct gaccaccacg     540
ctcggcaagg gcgtggtacg cgccaaggac acgcccaact tcatcgccaa ccgcgtcggc     600
atcttctcga tcctggccgt gtttgccgaa gccgagaagt tcggcatccc gttcgacgtg     660
gtcgatgacc tgaccggctc caagctgggc cgcgccaagt ccgccacctt ccgcaccgcg     720
gacgtggtcg gcctggacac catggcgcac gtgatcaaga ccatgcagga caccctgcac     780
gacgacccgt tcgcgccggt gtacaagacc ccggccgtac tcaagggcct ggtcgacgcg     840
ggcgcgctgg gccagaagac cggtgccggc ttctacaaga aggaaggcaa ggccatcaag     900
gtgctcgacg ccaagaccgg ccagtacgtc gatgccggca agaaggccga cgagatcgtg     960
gtgcgcatgc tgaagaagga tgcggccgag cgcatcaagc tgctgcgcga gtcgaccaac    1020
ccgcaggcgc agttcctgtg ggcggtgttc cgcgacgtgt tccactacat cgccgtctac    1080
ctggagcaga tcgcaggctc cgccgccgac atcgacctgg cgatccgctg gggcttcggc    1140
tggaactcgg gtccgttcga agactggcag tcggccggct ggaagcaggt ggccgagtgg    1200
gtcaaggaag acgtggaagc cggcaaggcg ctgtcggccg cgccgctgcc ggcatgggta    1260
ttcgagggcc cggtggcaga gaaccagggc gtgcacgccg ctgccggttc gtggtcgccg    1320
gcgacgcagt ccttcgtcgc gcgcagcaag ctgccggtgt accagcgcca ggcattccgt    1380
gccgcgatca agggcactgc cgcggccgat ccgcgcaagg ccggccgcac cgtcgaagag    1440
aacgacgccg tgcgcatctg ggtgagcgaa ggccaggacg acgtgctggt ggtctcgttc    1500
aagagcaaga tgaacaccat cgggccggac gtgatcgacg gcctgacccg cgccatcgac    1560
ctggccgaag ccggctacaa ggggctggtg gtgtggcagc cgacttcgct gcagctgggc    1620
gcaccgggtg gcccgttctc cgccggcgcc aacctggaag cggccatgcc cgccttcatg    1680
atgggcggcg ccaagggcat cgaaccgttc gtgaagaggt tccaggacgg catgatgcgc    1740
gtgaagtacg cctcggtgcc ggtggtgtcc gcggcgtccg gcattgcgct gggcggcggc    1800
tgcgagctga tgctgcattc ggcttcgcgc gtggccgcgc tggagacgta tatcggcctg    1860
gtcgaagtgg gcgtgggcct ggtgccggcc ggcggcggcc tgaaggaagc cgcgctggcg    1920
gccgcgcgcg cggcgcaggc cgcgggcagc accaacatcc tgcagttcct gaccagccgc    1980
ttccagagcg cggccatggc caaggtctcg gcctcggcgc tggaagcgcg ccagatgggc    2040
tacctgcagc cgtccgacaa gatcgtcttc aacgtgcacg agctgctgta cgtggcgcag    2100
aacgaagtgc gcgcgctggc cagcgccggc taccgcgcgc cgctgccgac gctggtcccg    2160
gtggccggcc gctcgggcat tgccaccatc aaggcatcgc tggtcaatat gcgcgacggc    2220
ggctttatct cgacgcacga cttcctgatc gccagccgca tcgccgaggc ggtgtgcggc    2280
ggcgacgtcg aggccggctc gctggtgagc gaggactggc tgctggcgct ggagcgcaag    2340
gcctttgtcg acctgctcgg caccggcaag acgcaggagc gcatcatggg catgctgcag    2400
accggcaagc cggtgcgtaa ctaa                                           2424

<210>  2
<211>  2082

<212>  DNA
<213>  Ralstonia eutropha (H16_A1526)

<400>  2
atgacagcgc agtatcaggt tcaggacggc gtagccgtca tcacgctcga caatccccct      60
gtcaacggcc tgggtcacag cacccggctt ggcatcgtcg aaggcatgac ccgtgcgctg     120
gacgatgccg ccgtcaaggc catcgtcatc accggcgctg gcaaggcttt ctcgggcggc     180
gccgacatcc gtgaattcaa tacgcccaag gccatgcagg agccgacgct gcactcggtg     240
atccgcgtgc tggaaggctc gtccaagccg gtcgtggccg ccgtgcactc ggtcgccatg     300
ggtggcggcc tggagctggc gctgggctgc aactaccgcg tggcgtccaa gggcgcccag     360
atcgcgctgc cggaagtgaa gctgggcctg ctgccgggcg ccggcggcac gcagcgcctg     420
ccgcgcgtga tcggcctgga ggccgcggcc aacatgatcg tgtcgggcac cccggtgctg     480
tccgagaagt ttgccggcac caagctgttc gatgaaatcg tcgacggcga cgtgctgccc     540
gccgcggtca agttcgcgca gaacgttggc gccgccaccg gcccgcatcc gaaggtgcgc     600
gacctgaagg tgcgccacga gaaccccgaa ggctacctgg gctttgcccg caataccgtg     660
gccgcgatgg ccaagaactt cccggcgccg ctgaagtgcc tggaagcggt ggccggctcg     720
ctcaagccgt tcgaacaggg cctgaagcaa gagcgcgagg gcttcctcta cctggttacc     780
acgccggaat cgcgcgcgct gcgccacgcc ttctttggcg agcgtgccgc cagcaagatc     840
cccgatgtgc ccgaaggcac gccgacccgc aagatcgaga aggtcgccgt gatcggcgcc     900
ggcaccatgg gcggcggcat cagcatgaac ttcctcaacg cgggcatccc ggtcaccatc     960
ctggaaacca agcaggaagc gctcgaccgc ggcgtcggca tcatccgcaa gaactacgag    1020
aacagcgcca agaagggcaa gctcacgcaa gagaaggtcg agcagcgcat gggcctgctc    1080
agcaccacgc tgtcctatga cgacctcaag gacgccgacc tgatcatcga ggccgtgttc    1140
gaagagatgg gcgtcaagga gaccgtgttc aagaagctgg atgaagtcgc caagcaaggc    1200
gcgatcctgg cctcgaacac ctccacgctg gacgtcaaca agatcgcctc gttcaccaag    1260
cgtccgcagg acgtggtcgg catgcatttc ttcagcccgg ccaatgtgat gaagctgctg    1320
gaagtggtgc gcggcgagaa gaccggcaag gacgtgctgg ccacggtcat gcaggtcggc    1380
aagaagatca agaagaccgc ggtggtgtcg ggcgtttgcg acggctttat cggcaaccgc    1440
atgatcgagc agtacagccg ccaggctggc tacctgctgg acgaaggcgc gctgccggag    1500
caggtcgaca aggccatcga gaagttcggc tttgccatgg gcccgttccg catgggcgac    1560
ctggccggca acgacatcgg ctgggccatc cgcaagcgcc gcgcggtgga caagccggag    1620
atccagtatt ccaagaccgc ggacctgctg tgcgagatgg gccgcttcgg ccagaagacc    1680
ggcgcgggct ggtacgacta caaggcgggc gaccgcaagc cgtacccgaa ccagcaggtc    1740
aacgacatga tcgtgcagca ctcgaaggac ctgggcatca cgcgccgcaa gatctctgac    1800
gaagagatcg tcgagcgcct ggtgttcgcg ctggtcaacg aaggcgccag gatcctggag    1860
gaaggcattg cttccaaggc ctcggatatc gacatggtgt acctgaccgg ctacggcttc    1920
ccgctgttcc gcggcggccc gatgctgtac gcggaccagg tcggcctgta caacgtggcg    1980
ctgtcgatga agcgctacgc caagggctac cacggtgaag cgtggcaggt cgcgccgctg    2040
ctgcagaagc tggcggacga aggcaagggc ttcaacggtt aa                        2082

<210>  3
<211>  2079
<212>  DNA
<213>  Ralstonia eutropha (H16_B0724)

<400>  3
atgaccgcca tcaacgccgt gaccgacctc acggtggacg ccgacatcgc cgtcctgagc      60
ctcgactcgc cccccgtcaa tgcactgtcc gccgccgtgc gcagcggcat ccgcgacggc     120
atggcgcgcg cgctcgccga cgatgccgtc aaggccgtgg tgctgatctg cgccggcaag     180
acctttgtcg cgggcgcgga catcaccgag tttggcaagc cgccggcggg cccgtcgctg     240
gcggaggtgc aggccatgat cgaggacgcg cccaagccgg tggtggctgc catccacggc     300
accgcgctcg gcggcgggct ggagctggcg ctggtgtgcc actaccgcat tgccacgcgc     360
tcggcaaagt gcgggctgcc ggaagtcaat ctcggcctgc tgccgggggc gggcggcacc     420
cagcggctgc cgcgtatcgt gggcccggaa caggcgctgg agatggtcac ctcgggcacg     480
cacgtggcgg ccagcgcagc ggcggagatg ggtctggtcg acctgctgac cgatgacgcc     540
tcgttgcgtg ccgacgcggt ggcgttcgca cgcaaggtgg tggccgagaa gcggcccctg     600
cgcaaggtgc gcgaatccga tgacaaggtg gcggccgcgc gcggcaagcc ggaactgttc     660
gctgccttcc gcaaggccaa tgcgcggcgc tttcgcggct tcgatgcgcc cgagttcaat     720
atccgctgca tcgaggcggc ggtggagctg ccattcgaag ccggcctgaa gcgcgagcgc     780
gagctgttca ccgaactgct caacggcacg cagtcggcgg cccagcgcta ctacttcttt     840
gcctcgcgcc aggtatggaa cgtgccggat gtgggagccg acacgcccgc ccttgcggtc     900
aggcgcatcg gcatcgtcgg cgcgggcacc atgggcggcg gcatcgccat gaacttcctc     960
aacgccggct atccggtgac catcgtcgag tcctcgcagc aggcgctgga ccgcggcctg    1020

aagacgatcc gggccaacta cgacaacacc gcgcgcaagg ggcgcctgag cgcggccgat 1080
gtcgaggccc gcatggcgct gctcacgccc acgctggacc tgcagctgct ggccgatgcc 1140
gacctgatca tcgaagcggt gttcgagaac atggaggtca agcaggagct gttcggcaag 1200
ctggaccgga tcgccaaagc gggcgccatc cttgccacca atacctcggc gctggacgtg 1260
aacgtgatcg cggccgcgag ctcgcgcccg gaggcggtca tcggcctgca tttcttctcg 1320
cccgccaacg tgatgaagct gctggaagtg gtgcgcggcg acaagaccgc aaagccggtg 1380
atccgcaccg cgatggaact cgcgcgcaag atcggcaagg tcgcggcgct ggtcggggta 1440
tgcccgggtt ttgtcggcaa ccgcatgctg gcccagcgcc agcgcgaggc ccagaagctg 1500
gtgctggacg gggcgctgcc gtgggacgtg gaccgcgtgc tgtacgactt cggctttccg 1560
atgggcccct tcgccatgag cgatctcgcc gggcttgacc ttggctgggt gcgcgagaag 1620
tccagcggct cgacgctgcg cgaggtgctg tgcgagatgg accgccgcgg gcagaaggcc 1680
ggcgccggct actacgacta cgacgaacag cgcaacgcga agccgtcgcc ggtggtcgag 1740
caggtcattc gcgacttcgc cgcgaaacag ggcaggaaca gccgcgtggt gtcggagcag 1800
gagatcctgg atcgctgcat ctatccgatg atcaacgaag gcgcgaagat cctgcaggaa 1860
ggcaaggcga ttcgcgcctc ggacatcgac gtgatctggg tgaacggcta cggctggccg 1920
gtgtatcgcg gcggcccgat gatctatgcc gacaccatcg ggctggacaa ggtgctggcg 1980
gcaatgcggc gctatgaggc gctgtatggc gcggacttcg cgccggcgcc actgctggag 2040
gaactggcgg cgcaggggcg caggttcgcc gatctctaa 2079

<210> 4
<211> 31
<212> DNA
<213> Artificial Sequence

<220>
<223> Oligonucleotide primer used in PCR for amplifying gene region
      including fadB1 gene

<400> 4
cccaagcttt cagcgcgaac cagtactcgg c 31

<210> 5
<211> 30
<212> DNA
<213> Artificial Sequence

<220>
<223> Oligonucleotide primer used in PCR for amplifying gene region
      including fadB1 gene
<400> 5
tgctctagag acgagcagaa gcggttgacg 30

<210> 6
<211> 31
<212> DNA
<213> Artificial Sequence

<220>
<223> Oligonucleotide primer used in PCR for amplifying gene region
      including fadB1 gene

<400> 6
gctggttcct ttggtgtcaa aagttgtctc g 31

<210> 7
<211> 32
<212> DNA
<213> Artificial Sequence

<220>
<223> Oligonucleotide primer used in PCR for amplifying gene region
      including fadB1 gene

<400>  7
taccgccaca tggttcagcc tggagctgag at                                    32

<210>  8
<211>  31
<212>  DNA
<213>  Artificial Sequence

<220>
<223>  Oligonucleotide primer used in PCR for amplifying gene region
       including fadB2 gene

<400>  8
cgcggatccg tgcagaacgt cagcttcaac t                                     31

<210>  9
<211>  39
<212>  DNA
<213>  Artificial Sequence

<220>
<223>  Oligonucleotide primer used in PCR for amplifying gene region
       including fadB2 gene

<400>  9
ccggaattcc cagaagcgct cgccgttcgc gcccaacgt                             39

<210>  10
<211>  30
<212>  DNA
<213>  Artificial Sequence

<220>
<223>  Oligonucleotide primer used in PCR for amplifying gene region
       including fadB2 gene

<400>  10
ggatcggttc tcctggattg aatcggtgtg                                       30

<210>  11
<211>  34
<212>  DNA
<213>  Artificial Sequence

<220>
<223>  Oligonucleotide primer used in PCR for amplifying gene region
      including fadB2 gene

<400>  11
ggcagcaagg gctgcctcgg ccggccggtg ccat                                  34

<210>  12
<211>  31
<212>  DNA
<213>  Artificial Sequence

<220>
<223>  Oligonucleotide primer used in PCR for amplifying gene region
       including fadB' gene

<400>  12
cccaagcttg cagatcgacc gcgagttgtc g                                     31

<210>  13
<211>  33
<212>  DNA
<213>  Artificial Sequence

<220>
<223>  Oligonucleotide primer used in PCR for amplifying gene region
       including fadB′ gene

<400>  13
ctagtctaga caaaggcctc gttcagttcg atc                                    33

<210>  14
<211>  31
<212>  DNA
<213>  Artificial Sequence

<220>
<223>  Oligonucleotide primer used in PCR for amplifying gene region
       including fadB′ gene

<400>  14
gctggttcct ttggtgtcaa aagttgtctc g                                      31

<210>  15
<211>  32
<212>  DNA
<213>  Artificial Sequence

<220>
<223>  Oligonucleotide primer used in PCR for amplifying gene region
       including fadB′ gene
<400>  15
taccgccaca tggttcagcc tggagctgag at                                     32

## Description

### TECHNICAL FIELD

The present invention relates to a method for microbially producing poly(3-hydroxybutyrate-co-3-hydroxyhexanoate), one of the copolyesters that can be microbially biodegradable and excellently biocompatible, using a vegetable oil as a basic raw material.

### BACKGROUND ART

While petrochemical plastics, an essential material in modern society, are inexpensive and easily processed, they are posing a disposal problem due to their persistent nature. Thus, polyhydroxyalkanoates (PHAs) intracellularly accumulated as an energy source by various microorganisms are expected as a plastic material having a small environmental burden that uses biomass instead of petroleum as a raw material and that is biodegradable. Poly(3-hydroxybutyrate) (hereinafter referred to as "P(3HB)") is a representative PHA that is biosynthesized by various microorganisms. However, because of its physical properties of being hard and brittle, P(3HB) may be hard to be put into practical use.

On the other hand, poly((R)-3-hydroxybutyrate-co-(R)-3-hydroxyhexanoate) copolymer (hereinafter referred to as "P(3HB-co-3HHx)") synthesized from a vegetable oil by *Aeromonas caviae (A. caviae*) exhibits an excellent property of being flexible. Furthermore, since this copolymer may exhibit a wide variety of physical properties that may be applicable to hard polymers to soft polymers depending on the 3HHx composition, its application into a variety of uses may be expected (Non-patent document 1).

So far, A. caviae has been known to use a vegetable oil as a raw material to synthesize P(3HB-co-3HHx) in which the 3HHx (hydroxyhexanoate) fraction is 10-20 mol%. However, its intracellular content is as low as about 15% by weight, which is difficult to be put into practical production (Patent Document 1, Patent Document 2, Non-patent document 1). In order to realize practical application of microbial polyesters, a technology is required that enables the efficient production of copolymers having a diverse range of compositions that realize various physical properties. Attempts have previously been made to search for methods for producing recombinant strains capable of accumulating P(3HB-co-3HHx) having a high 3HHx fraction that also demonstrate high proliferation ability, and the introduction of a PHA synthase mutant enzyme gene derived from *Aeromonas caviae* into a chromosome of *Cupriavidus necator,* a hydrogen bacterium exhibiting highly efficient production of P(3HB), to deactivate the supply enzyme of 3HB monomer in the form of β-ketothiolase gene or acetoacetyl-CoA reductase, followed by subjecting to modification by introducing a R-hydratase gene, which converts an intermediate of the fatty acid β-oxidation pathway in the form of 2-enoyl-CoA to R-3HA-CoA, has been disclosed (Patent Document 3, Patent Document 4, Non-Patent Document 2).

On the other hand, although there have been hardly any reports describing the fatty acid β-oxidation pathway of *Cupriavidus necator,* a strain obtained by deleting two regions consisting of H16_A0459-A0464 and H16_A1526-A1531 on chromosome 1 of *Cupriavidus necator* has been reported to exhibit a remarkable decrease in growth in vegetable oil (Non-Patent Document 3). However, there have been no examples of reports of applying this finding to the production or compositional control of PHA copolymers.

### Prior Art Documents

### Patent Documents

Patent Document 1: Japanese Unexamined Patent Publication No. H5-93049
Patent Document 2: Japanese Unexamined Patent Publication No. H7-265065
Patent Document 3: Japanese Patent No. 3062459
Patent Document 4: International Publication No. WO 2011/105379

### Non-Patent Documents

Non-Patent Document 1: Doi, et al., Macromolecules, 28, 4822-4828 (1995)
Non-Patent Document 2: Fukui, et al., Appl. Microbiol. Biotechnol., 49, 333-336 (1998)
Non-Patent Document 3: Brigham, et al., J. Bacteriol., 192, 5454-5464 (2010)

### DISCLOSURE OF THE INVENTION

### Means for Solving the Problems

The inventors of the present invention have found that a microorganism that produces poly(3-hydroxybutyrate-co-3- hydroxyhexanoate) ("P(3HB-co-3HHx)") can be produced by disrupting the gene of a bifunctional enzyme in the form of FadB, which catalyzes the hydration reaction of 2-enoyl-CoA and subsequently the dehydrogenation reaction of (S)-3-hydroxyacyl-CoA in the fatty acid β-oxidation pathway, in a recombinant strain of *Cupriavidus necator* imparted with the ability to produce P(3HB-co-3HHx), thereby leading to completion of the present invention.

According to the present invention, a method is provided for producing P(3HB-co-3HHx) having a high 3HHx fraction at a high accumulation rate using vegetable oil and/or fatty acid as raw material by disrupting a gene encoding 2-enoyl-CoA hydratase or a gene encoding 3-hydroxyacyl-CoA dehydrogenase on a chromosome of recombinant *Cupriavidus necator* imparted with the ability to produce P(3HB-co-3HHx).

Namely, the present invention is as described below.
[1] A method for producing poly(3-hydroxybutyrate-co-3-hydroxyhexanoate), comprising:
   (a) providing a recombinant *Cupriavidus necator* strain imparted with the ability to produce poly(3-hydroxybutyrate-co-3-hydroxyhexanoate),
   (b) completely or partially deleting, modifying or disrupting at least one gene encoding 2-enoyl-CoA hydratase or at least one gene encoding 3-hydroxyacyl-CoA dehydrogenase on a chromosome of the strain to suppress expression of the gene, and
   (c) growing the strain in medium containing vegetable oil as a carbon source; wherein,
   the 3-hydroxyhexanoate composition of poly(3-hydroxybutyrate-co-3-hydroxyhexanoate) is 1 mol% to 20 mol% and the strain internal accumulation rate is 50% by weight to 90% by weight.
[2] The method described in [1] above, wherein the recombinant *Cupriavidus necator* strain is a NSDG strain, a NSDGΔA strain, a MF01 strain, a MF03 strain or a MF03-J1 strain.
[3] The method described in [1] and [2] above, wherein the gene encoding 2-enoyl-CoA hydratase or the gene encoding 3-hydroxyacyl-CoA dehydrogenase is selected from the group consisting of fadB1 (SEQ ID NO: 1), fadB2 (SEQ ID NO: 2), fadB' (SEQ ID NO: 3), and combinations thereof.
[4] The method described in [1] to [3] above, wherein the suppression of gene expression is gene disruption.
[5] A method for producing a recombinant *Cupriavidus necator* strain that produces poly(3-hydroxybutyrate-co-3-hydroxyhexanoate) having an improved 3-hydroxyhexanoate fraction, comprising:
   (a) providing a recombinant *Cupriavidus necator* strain imparted with the ability to produce poly(3-hydroxybutyrate-co-3-hydroxyhexanoate),
   (b) constructing a vector for completely or partially deleting, modifying or disrupting at least one gene encoding 2-enoyl-CoA hydratase or at least one gene encoding 3-hydroxyacyl-CoA dehydrogenase on a chromosome of the strain, and
   (c) introducing the vector constructed in (b) into the strain of (a) to suppress function of the gene by disrupting or modifying the gene by homologous recombination.
[6] The method described in [5] above, wherein the recombinant *Cupriavidus necator* strain is a NSDG strain, a NSDGΔA strain, a MF01 strain, a MF03 strain or a MF03-J1 strain.
[7] The method described in [5] and [6] above, wherein the gene encoding 2-enoyl-CoA hydratase or the gene encoding 3-hydroxyacyl-CoA dehydrogenase is selected from the group consisting of fadB1 (SEQ ID NO: 1), fadB2 (SEQ ID NO: 2), fadB' (SEQ ID NO: 3), and combinations thereof.
[8] The method described in [5] to [7] above, wherein the suppression of gene expression is gene disruption.

### Effects of the Invention

A strain can be produced that produces poly(3-hydroxybutyrate-co-3-hydroxyhexanoate) (P(3HB-co-3HHx)) having a high 3HHx fraction while maintaining high proliferation ability and high PHA production ability by completely or partially deleting, modifying or disrupting at least one gene encoding 2-enoyl-CoA hydratase or at least one gene encoding 3-hydroxyacyl-CoA dehydrogenase on a chromosome of a microorganism producing P(3HB-co-3HHx).

### BRIEF DESCRIPTION OF THE DRAWINGS

FIG. 1 shows the biosynthesis pathway of poly(3-hydroxybutyrate-co-3-hydroxyhexanoate) in a microorganism.
FIG. 2 shows an FadB homolog found in a *Cupriavidus necator* strain.

### MODE FOR CARRYING OUT THE INVENTION

The following provides a detailed description of preferred embodiments for explaining the present invention.

As described above, the present invention provides a method for producing poly(3-hydroxybutyrate-co-3-hydroxyhexanoate), the method comprising:
(a) providing a recombinant *Cupriavidus necator* strain imparted with the ability to produce poly(3-hydroxybutyrate-co-3-hydroxyhexanoate),
(b) completely or partially deleting, modifying or disrupting at least one gene encoding 2-enoyl-CoA hydratase or at least one gene encoding 3-hydroxyacyl-CoA dehydrogenase on a chromosome of the strain to suppress expression of the gene, and
(c) growing the strain in medium containing vegetable oil as a carbon source; wherein,
   the 3-hydroxyhexanoate fraction is 1 mol% to 20 mol% and the strain internal accumulation rate is 50% by weight to 90% by weight.

### (1) Recombinant Cupriavidus necator Strain

A recombinant *Cupriavidus necator* strain imparted with the ability to produce P(3HB-co-3HHx) is preferable for the microorganism used in the production method of the present invention. Here, a "recombinant *Cupriavidus necator* strain imparted with the ability to produce P(3HB-co3HHx)" refers to a strain in which a broadly substrate-specific PHA synthase, capable of using 3HHx-CoA having six carbon atoms as a substrate (such as PHA synthase derived from an *Aeromonas caviae* strain or a gene of a mutant enzyme thereof), is introduced into a strain from which PHA synthase inherently possessed by a *Cupriavidus necator* strain has been removed by mutation or gene disruption for the purpose of biosynthesis of P(3HB-co-3HHx), or a strain in which PHA synthase gene inherently possessed by a *Cupriavidus necator* strain has been substituted on a chromosome thereof with a broadly substrate-specific PHA synthase or a gene of a mutant enzyme thereof, and the strain has the ability to synthesize P(3HB-co-3HHx) that cannot be synthesized by a wild strain of *Cupriavidus necator* by the action of a broadly substrate-specific PHA synthase. Although there are no particular limitations on the aforementioned recombinant *Cupriavidus necator* strain imparted with the ability to produce P(3HB-co-3HHx) provided it is a strain that has the aforementioned characteristics, examples thereof include a NSDG strain, a NSDGΔA strain, a MF01 strain, a MF03 strain and a MF03-J1 strain. Here, "NSDG strain" refers to a transformant in which a PHA synthase mutant enzyme gene in the form of phaC_{NSDG} has been introduced into a chromosome of a type of hydrogen bacterium *Cupriavidus necator* in the form of a H16 strain. "NSDGΔA strain" refers to a transformant in which a β-ketothiolase gene in the form of phaA_{Cn} has been deleted in the aforementioned NSDG strain. "MF01 strain" refers to a transformant in which phaA_{Cn} of the aforementioned NSDG strain has been substituted with a broadly substrate-specific β-ketothiolase gene in the form of bktB_{Cn}. "MF03 strain" refers to a transformant in which phaA_{Cn} of the aforementioned NSDG strain has been substituted with an (R)-specific enoyl-CoA hydratase (R-hydratase) gene (phaJ_{Ac}) derived from *Aeromonas caviae.* In addition, "MF03-J1 strain" refers to a transformant in which phaA (β-ketothiolase gene) of the aforementioned NSDG strain has been substituted with an R-hydratase gene (phaJ1_{Cn}) derived from *Cupriavidus necator* (refer to the aforementioned Patent Document 4 for further details). The aforementioned five types of H16 mutant strains can be produced using ordinary genetic engineering techniques based on sequence information on genes encoding PHA synthase enzyme of *Cupriavidus necator.*

### (2) Gene Encoding 2-Enoyl-CoA Hydratase and Gene Encoding 3-Hydroxyacyl-CoA Dehydrogenase

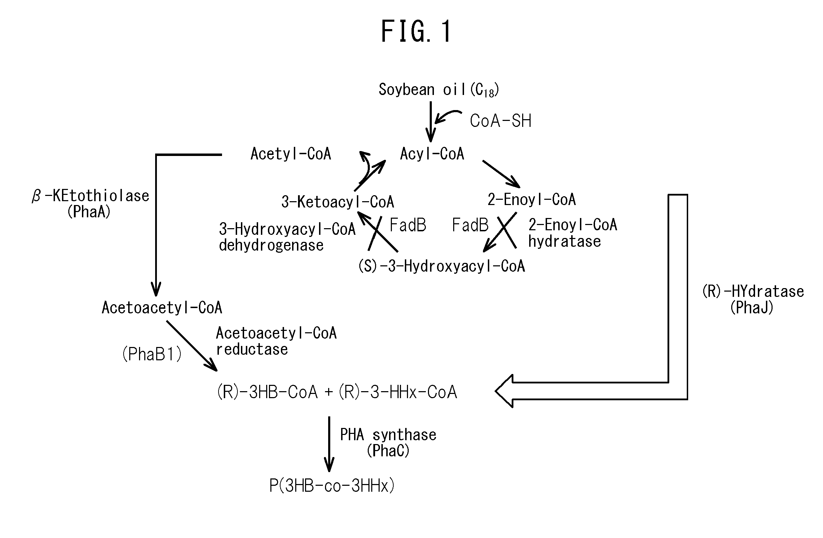

The "2-enoyl-CoA hydratase" used in the production method of the present invention refers to an enzyme that catalyzes the hydration reaction from an intermediate of the fatty acid β-oxidation pathway in the form of 2-enoyl-CoA to (S)-3-hydroxyacyl-CoA. On the other hand, the "3-hydroxyacyl-CoA dehydrogenase" used in the production method of the present invention refers to an enzyme that catalyzes the dehydrogenation reaction from an intermediate of the fatty acid β-oxidation pathway in the form of the aforementioned (S)-3-hydroxyacyl-CoA to 3-ketoacyl-CoA. A plurality of the genes that encode these enzymes is known to be present in *Cupriavidus necator* strains. In addition, according to the present invention, the production of (R)-3HHx-CoA (monomer of P(3HB-co-3HHx)) from 2-enoyl-CoA by (R)-hydratase (PhaJ) can be promoted by interrupting the pathway for forming (S)-3-hydroxyacyl-CoA from 2-enoyl-CoA (by 2-enoyl-CoA hydratase) followed by forming 3-ketoacyl-CoA (by 3-hydroxyacyl-CoA dehydrogenase) in the fatty acid β-oxidation pathway in a recombinant *Cupriavidus necator* strain imparted with the ability to produce P(3HB-co-3HHx) (see FIG. 1). Accordingly, according to the present invention, a recombinant *Cupriavidus necator* strain having further enhanced ability to produce P(3HB-co-3HHx) can be produced by completely or partially deleting, modifying or disrupting (which may be simply referred to as "disrupting") at least one gene encoding the aforementioned 2-enoyl-CoA hydratase or at least one gene encoding the aforementioned 3-hydroxyacyl-CoA dehydrogenase on a chromosome of a recombinant *Cupriavidus necator* strain imparted with the ability to produce P(3HB-co-3HHx) to suppress expression of that gene. Thus, either of the aforementioned two genes may be targeted for disruption provided a strain can be obtained that has enhanced ability to produce the desired P(3HB-co-3HHx) by disrupting any one of the aforementioned two genes. According to the present invention, a target strain can be preferably produced by completely or partially deleting, modifying or disrupting, and more preferably by disrupting, both of the aforementioned two genes. Furthermore, the nucleic acids (genes) encoding the aforementioned enzymes used in the production method of the present invention include single-stranded or double-stranded DNA and an RNA complement thereof. DNA includes naturally-derived DNA, recombinant DNA, chemically synthesized DNA, DNA amplified by PCR and combinations thereof. DNA is preferable for the nucleic acid used in the present invention. As is widely known, although codons are subject to degeneration and there are amino acids for which there are a plurality of base sequences that encode a single amino acid, nucleic acids having any base sequence are included within the scope of the present invention provided the base sequence is that of a nucleic acid that encodes the aforementioned enzymes.

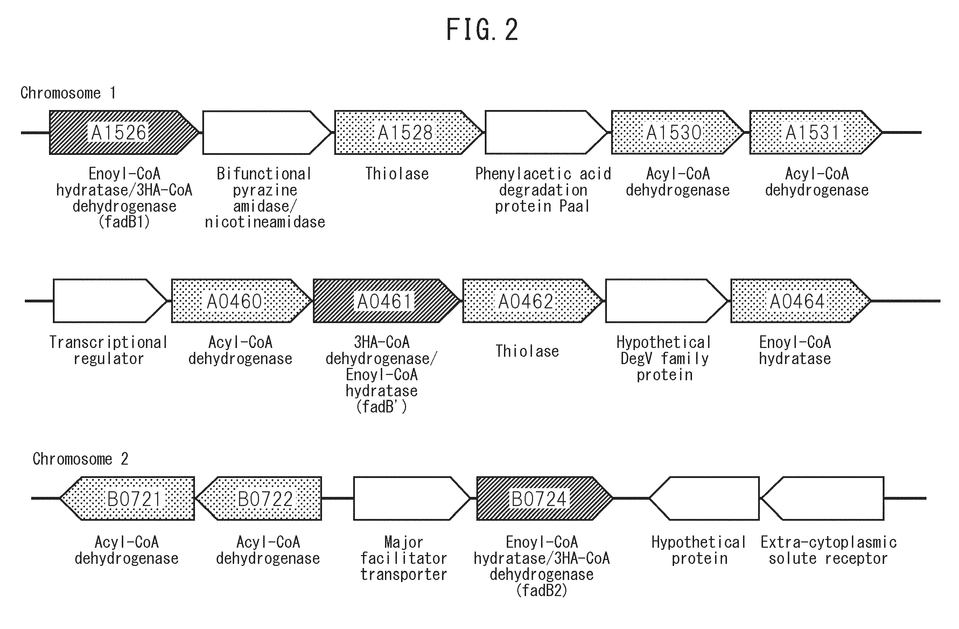

According to the present invention, although there are no particular limitations thereon, the aforementioned two genes allow the obtaining of a strain having enhanced ability to produce the desired P(3HB-co-3HHx) by, for example, completely or partially deleting, modifying or disrupting both genes that encode a bifunctional enzyme in the form of FadB that catalyzes a hydration reaction of 2-enoyl-CoA followed by a dehydrogenation reaction of (S)-3-hydroxyaryl-CoA. Here, the FadB gene is identified as H16_A1526 (485753-488176 of chromosome 1 (NC_008313) : SEQ ID NO: 1) (also referred to as "fadB1" in the present description), H16_B0724 (817223-819301 of chromosome 2 (NC_008314) : SEQ ID NO: 2) (also referred to as "fadB2" in the present description) and H16_A0461 (485753-488176 of chromosome 1 (NC_008313) :SEQ ID NO: 3) (also referred to as "fadB'" in the present description) on a chromosome of *Cupriavidus necator* (see FIG. 2).

### (3) Construction of Vector and Production of Recombinant Microorganism

As described above, the recombinant microorganism used in the production method of the present invention is obtained by completely or partially deleting, modifying or disrupting at least one gene that encodes 2-enoyl-CoA hydratase or at least one gene that encodes 3-hydroxyaryl-CoA dehydrogenase on a chromosome of a recombinant *Cupriavidus necator* strain imparted with the ability to produce P(3HB-co-3HHx). Here, "deletion" of a gene in the present description refers to removing one or more bases from the base sequence of a gene encoding the aforementioned enzymes, and refers to the loss of a portion, and preferably all, of the activity of those enzymes as a result thereof. Furthermore, in the production method of the present invention, the base sequence of a gene that encodes the aforementioned enzymes may be completely deleted. In addition, "modification" of a gene in the present description refers to suppressing the activity of the aforementioned enzymes or deactivating the enzymes by inducing a deletion, substitution or addition of a base in the base sequence of a gene that encodes the aforementioned enzymes. On the other hand, "disruption" in the present description refers to causing the function of a gene that encodes the aforementioned enzymes to be lost by inserting, for example, a foreign gene into the base sequence of a gene that encodes those enzymes. Here, in the present description, in the case of using for the purpose of preventing a target enzyme from functioning, the terms "deletion", "modification" and "disruption" may be used interchangeably without any strict distinction.

A method consisting of introducing a vector into cells is typically used as a method for causing the loss of function of a target gene on a chromosome of a cell. For example, disruption of a gene that encodes the aforementioned enzymes can be induced in the case of arranging a fragment of genomic DNA together with an intrinsic homologous sequence and substituting the gene and the vector on a chromosome by homologous recombination. In one aspect, this type of disruption is preferably complete disruption and there is preferably no significant expression of the aforementioned enzymes. According to the present invention, a gene substitution vector incorporated in a vector for homologous recombination of a gene that encodes a target enzyme is provided as a constructed vector used to delete, modify or disrupt a gene that encodes the aforementioned enzymes on a chromosome of a microorganism. Here, an example of a method used to incorporate a gene in a vector is described in Sambrook, J. et al., Molecular Cloning: A Laboratory Manual (3rd edition), Cold Spring Harbor Laboratory, 1, 1 (2001). For the sake of simplicity, a commercially available ligation kit (such as that manufactured by Toyobo Co., Ltd.) can also be used.

A vector can be prepared by ligating a desired gene to a recombination vector (such as plasmid DNA), able to be easily acquired in the art, in accordance with ordinary methods. In the case of using a vector for the purpose of disrupting gene expression, a selection marker simultaneously incorporated on the chromosome (such as neomycin resistance gene or kanamycin resistance gene) is preferably introduced in addition to the desired gene. Although there are no particular limitations thereon, vector pK18mobsacB (Schafer, et al., Gene, 145, 69-73 (1994)) or pJQ200 (Quandt, J. and Hynes, M.P., "Versatile suicide vectors which allow direct selection for gene replacement in gram-negative bacteria", Gene, 127, 15-21 (1993)) can be used for the vector used in the method for producing P(3HB-co-3HHx) of the present invention for the purpose of substituting a gene encoding a target enzyme on a chromosome of a microorganism with a foreign gene (such as a gene encoding a target enzyme combined with an antibiotic resistance gene). Moreover, since a strain introduced with the aforementioned pK18mobsacB vector accumulates toxic polysaccharides in the cells thereof using sucrose as substrate due to the presence of levansucrase encoded by sacB in the vector, a strain from which a gene encoding a target enzyme has been knocked out can be selectively obtained by culturing the strain in medium containing sucrose.

A person skilled in the art could select, as appropriate, a restriction end so as to be compatible with a recombinant vector, and, furthermore, in order to express the desired protein, could select, as appropriate, a recombinant vector suitable for the host cells. By suitably arranging or introducing regions (as needed, an autonomous origin of replication, a transconjutation region, a selection marker, and the like) that serve to trigger homologous recombination between the gene for use in the present invention and the gene of the host cell of interest, such a vector has been or should be constructed so that the nucleic acid can be suitably recombined.

Generally, introduction of a recombinant vector into host cells (transformation) can be carried out using a known method. For example, in the case of bacteria (such as *Escherichia coli* or *Bacillus subtilis),* examples of methods that can be used include the method of Cohen, et al. (Proc. Natl. Acad. Sci. USA, 69, 2110 (1972)), the protoplast method (Mol. Gen. Genet., 168, 111 (1979)), the competent cell method (J. Mol. Biol., 56, 209 (1971)), the calcium chloride method and electroporation. In addition, a conjugal transfer method can be used in the case of introducing an expression vector into bacteria belonging to the genus Ralstonia, Alcaligenes or Pseudomonas (J. Bacteriol., 147, 198 (1981)). Simply speaking, this conjugal transfer method utilizes the property of cells of allowing a chromosomal genome or plasmid to migrate from one cell to another cell due to contact between cells, and for example, and is a means for allowing gene introduction by a series of steps that begin with conjugation between a donor organism introduced with a self-transmissible plasmid that retains a target DNA and an acceptor organism that does not have that plasmid, followed by formation of a bridge between both organisms and subsequent replication and transfer of the plasmid, completion of DNA synthesis and isolation of the cells. As described above, the host cells used in the production method of the present invention are preferably those of a recombinant *Cupriavidus necator* strain imparted with the ability to produce P(3HB-co-3HHx). The host cells are more preferably those of a NSDG strain, a NSDGΔA strain, a MF01 strain, a MF03 strain or a MF03-J1 strain. Furthermore, in Examples 6 and 7 to be described below, a prepared gene recombinant vector (pK18msΔfadB1, pK18msΔfadB2 or pK18msΔfadB') was introduced into the aforementioned strain by conjugal transfer, and the recombinant strain, in which the vector was inserted into a chromosome by single-cross homologous recombination, was isolated using kanamycin resistance as an indicator. In addition, in the case of inserting for the purpose of disrupting gene expression of a target enzyme, for example, there are no particular limitations on the location where the foreign gene is introduced into a chromosome provided it is a location where the foreign gene is substituted with the gene of the target enzyme by the introduced vector by homologous recombination.

### (4) Synthesis of Copolymerized Polyester

Polyester synthesis is carried out by culturing the previously described recombinant *Cupriavidus necator* strain imparted with the ability to produce P(3HB-co-3HHx) (such as a NSDG strain, a NSDGΔA strain, a MF01 strain, a MF03 strain or a MF03-J1 strain) in a prescribed medium containing vegetable oil, allowing copolymerized polyester to be produced and accumulate in the cultured cells (such as in cell bodies) or culture (such as in the medium), and collecting the target copolymerized polyester from the cultured cells or culture. Furthermore, the method used to culture transformants in the production method of the present invention is normally carried out in accordance with a method used to culture host cells.

Examples of media in the case of using a recombinant *Cupriavidus necator* strain for the host include media obtained by adding a vegetable oil that can be assimilated by the microorganisms and in which restrictions have been placed on any of the nitrogen source, inorganic salts or other nutrient sources. Typically, a desired copolymerized polyester can be prepared by culturing aerobically at a medium temperature within the range of 25°C to 27°C for 1 to 10 days to allow copolymerized polyester to be produced and accumulate in the bacterial cells followed by the recovery and purification thereof.

The parent strain of the transformant used in the production method of the present invention in the form of *Cupriavidus necator* is known to be a bacterium that is able to grow using vegetable oil as a carbon source. Here, a commercially available vegetable oil can typically be used as a usable vegetable oil, and there are no particular limitations on the supply source thereof. The vegetable oil is preferably natural oil such as soybean oil, corn oil, soybean oil, safflower oil, sunflower oil, olive oil, coconut oil, palm oil, rapeseed oil, fish oil, whale oil, pork lard or beef tallow. Fatty acids obtained therefrom may also be used. In addition, according to the present invention, a transformant of the present invention can be cultured under culturing conditions similar to those of the parent strain in the form of *Cupriavidus necator,* and although the concentration of vegetable oil in the medium is preferably 0.1% to 5%, it can be suitably adjusted by a person skilled in the art.

In addition, a nitrogen source or inorganic substance may also be added to the medium as necessary. Examples of nitrogen sources include ammonia, ammonium salts such as ammonium chloride, ammonium sulfate or ammonium phosphate, peptone, beef extract, yeast extract and corn stiplica. Examples of inorganic substances include potassium dihydrogen phosphate, dipotassium hydrogen phosphate, magnesium phosphate, magnesium sulfate and sodium chloride.

Shake culturing is normally used for culturing and is preferably carried out for at least one day at 25°C to 37°C under aerobic conditions after inducing gene expression. An antibiotic such as kanamycin or ampicillin may be added to the medium. In addition, a gene expression inducer such as arabinose, indole acrylic acid (IAA) or isopropyl-β-D-thiogalactopyranoside (IPTG) can also be used. A person skilled in the art is able to suitably select culturing conditions or gene expression induction conditions and the like for enabling expression of a desired gene.

### (5) Purification and Structural Analysis of Copolymerized Polyester

In the present invention, the copolymerized polyester can be purified as follows: the transformant is recovered from the medium by centrifugal separation followed by washing with distilled water and drying or freeze-drying. Subsequently, the dried transformant is suspended in chloroform and stirred for a prescribed amount of time at room temperature to extract the copolymerized polyester. The suspension may be heated as necessary in the extraction stage. After removing the residue by filtration and adding methanol to the supernatant to precipitate the copolymerized polyester, the supernatant is removed by filtering or centrifuging the precipitate followed by drying the precipitate to obtain the purified copolymerized polyester.

Means used to confirm that the resulting copolymerized polyester is the desired product include, but are not limited to, methods using nuclear magnetic resonance (NMR) or gas chromatography. According to the present invention, the monomer unit ratio of the copolymerized polyester, namely the ratio of 3-hydroxybutyrate (3HB) to 3-hydroxyhexanoate (3HHx), can be suitably adjusted by changing gene expression conditions, culturing conditions and the like. The 3HHx fraction of the copolymerized polyester obtained according to the production method of the present invention is preferably 1 mol% to 20 mol%, more preferably 1 mol% to 15 mol%, and even more preferably 5 mol% to 12 mol%. The term "mol%", when used in the present description, refers to the value obtained by dividing the number of moles of a certain component by the sum of the number of moles of each component in a multicomponent system. In addition, the 3HHx fraction can be calculated by b x 100/(a + b) when the number of 3HB monomer units and the number of 3HHx monomer units in P(3HB-co-3HHx) are defined as "a" and "b", respectively. As indicated in Example 7 to be subsequently described, the copolymerized polyester produced according to the present invention accumulates in bacterial cells at 50% by weight to 90% by weight, and preferably 70% by weight to 90% by weight, per dry mass of the bacterial cells, and the 3HHx fraction is increased by about 1 mol% in comparison with copolymerized polyester produced by conventional methods.

### Examples

The following provides a more detailed explanation of the present invention based on examples thereof. The present invention is naturally not limited to the following examples.

### Example 1 Recombinant Cupriavidus necator Strains Used

In the following example, recombinant strains of *Cupriavidus necator* imparted with the ability to produce poly(3-hydroxybutyrate-co-3-hydroxyhexanoate) in the form of a NSDG strain, a NSDGΔA strain, a MF01 strain, a MF03 strain and a MF03-J1 strain were used as hosts for genetic recombination. "NSDG strain" is a transformant in which a PHA synthase mutant enzyme gene in the form of phaC_{NSDG} has been introduced into a chromosome of a type of hydrogen bacterium *Cupriavidus necator* in the form of strain H16, and "NSDGΔA strain" is a transformant in which a β-ketothiolase gene in the form of phaA_{Cn} has been deleted in the aforementioned strain NSDG. On the other hand, "MF01 strain" is a transformant in which phaA_{Cn} of the aforementioned strain NSDG has been substituted with a broadly substrate-specific β-ketothiolase gene in the form of bktB_{Cn}, "MF03 strain" is a transformant in which phaA_{Cn} of the aforementioned strain NSDG has been substituted with an R-hydratase gene (phaJ_{Ac}) derived from *Aeromonas caviae,* and "MF03-J1 strain" is a transformant in which phaA of the aforementioned strain NSDG has been substituted with an R-hydratase gene (phaJ1_{cn}) derived from *Cupriavidus necator.* Each of the strains was produced using ordinary genetic engineering techniques with reference to Patent Document 4.

### Example 2 FadB Gene in Cupriavidus necator Strain H16

Genes encoding a bifunctional enzyme that catalyzes the hydration reaction of 2-enoyl-CoA and the dehydrogenation reaction of (S)-3-hydroxyacyl-CoA in the form of FadB were targeted for disruption. FadB genes were identified in chromosomes of *Cupriavidus necator* as H16_A1526 (485753-488176 of chromosome 1 (NC_008313) :SEQ ID NO: 1) (also referred to as "fadB1" in the present description), H16_B0724 (817223-819301 of chromosome 2 (NC_008314) : SEQ ID NO: 2) (also referred to as "fadB2" in the present description) and H16_A0461 (485753-488176 of chromosome 1 (NC_008313) :SEQ ID NO: 3) (also referred to as "fadB'" in the present description) (see FIG. 2).

### Example 3 Production of Vectors for Disruption of fadB1

Gene fadB1 encoding FadB (SEQ ID NO: 1) and approximately 1 kbp regions upstream and downstream therefrom were amplified by PCR using genomic DNA of *Cupriavidus necator* strain H16 (NC_008313) as template and using the oligonucleotides of Sequence 1 and Sequence 2 indicated below as primers. PCR was carried out for 30 cycles consisting of reacting for 20 seconds at 98°C, 15 seconds at 65°C and 3 minutes at 68°C using KOD Plus (Toyobo Co., Ltd.). The amplified fragment was purified with a DNA purification kit (Promega Corp.).
Sequence 1: CCCAAGCTTTCAGCGCGAACCAGTACTCGGC (SEQ ID NO: 4)
   (Underline indicates HindIII restrictase site)
Sequence 2: TGCTCTAGAGACGAGCAGAAGCGGTTGACG (SEQ ID NO: 5)
   (Underline indicates XbaI restrictase site)

Subsequently, the 5'-terminal was phosphorylated by T4 kinase (Toyobo Co., Ltd.), and ligated with pUC118 (Takara Bio Inc.), subjected to HincII cleavage and alkaline phosphatase treatment, with Ligation High (Toyobo Co., Ltd.). A fragment was then amplified containing an approximately 1 kbp region upstream from fadB1, a pUC118 vector region and an approximately 1 kbp region downstream from fadB1 by inverse PCR using the resulting plasmid as template and using oligonucleotides of the following Sequence 3 and Sequence 4 as primers. PCR was carried out for 30 cycles consisting of reacting for 20 seconds at 98°C, 15 seconds at 65°C and 3 minutes at 68°C using KOD Plus. The amplified fragment was purified with a DNA purification kit.
Sequence 3: GCTGGTTCCTTTGGTGTCAAAAGTTGTCTCG (SEQ ID NO: 6)
Sequence 4: TACCGCCACATGGTTCAGCCTGGAGCTGAGAT (SEQ ID NO: 7)

Subsequently, the 5'-terminal was phosphorylated by T4 kinase and self-ligated with Ligation High. The resulting vector was cleaved with HindIII and XbaI to isolate a fragment in which the upstream region and downstream region of fadB1 were ligated. fadB1 disruption vector pK18msΔfadb1 was obtained by ligating this fragment with pK18mobSacB cleaved with HindIII and XbaI.

### Example 4 Production of Vector for fadB2 Disruption

Gene fadB2 encoding FadB (SEQ ID NO: 2) and approximately 1 kbp regions upstream and downstream therefrom were amplified by PCR using genomic DNA of *Cupriavidus necator* strain H16 (NC_008314) as template and using the oligonucleotides of Sequence 5 and Sequence 6 indicated below as primers. PCR was carried out for 30 cycles consisting of reacting for 20 seconds at 98°C, 15 seconds at 65°C and 3 minutes at 68°C using KOD Plus. The amplified fragment was purified with a DNA purification kit.
Sequence 5: CGCGGATCCGTGCAGAACGTCAGCTTCAACT (SEQ ID NO: 8)
   (Underline indicates BamHI restrictase site)
Sequence 6: CCGGAATTCCCAGAAGCGCTCGCCGTTCGCGCCCAACGT (SEQ ID NO: 9) (Underline indicates BamHI restrictase site)

Subsequently, the 5'-terminal was phosphorylated by T4 kinase, and ligated with pUC118, subjected to HincII cleavage and alkaline phosphatase treatment, with Ligation High. A fragment was then amplified containing an approximately 1 kbp region upstream from fadB2, a pUC118 vector region and an approximately 1 kbp region downstream from fadB2 by inverse PCR using the resulting plasmid as template and using oligonucleotides of the following Sequence 7 and Sequence 8 as primers. PCR was carried out for 30 cycles consisting of 20 seconds at 98°C, 15 seconds at 65°C and 3 minutes at 68°C using KOD Plus. The amplified fragment was purified with a DNA purification kit.
Sequence 7: GGATCGGTTCTCCTGGATTGAATCGGTGTG (SEQ ID NO: 10)
Sequence 8: GGCAGCAAGGGCTGCCTCGGCCGGCCGGTGCCAT (SEQ ID NO: 11)

Subsequently, the 5'-terminal was phosphorylated by T4 kinase and self-ligated with Ligation High. The resulting vector was cleaved with BamHI to isolate a fragment in which the upstream region and downstream region of fadB2 were ligated. fadB2 disruption vector pK18msΔfadB2 was obtained by ligating this fragment with pK18mobSacB that had been cleaved with BamHI and in which the 5'-terminal had been dephosphorylated with alkaline phosphatase (Toyobo Co., Ltd.).

### Example 5 Production of Vector for fadB' Disruption

Gene fadB' encoding FadB (SEQ ID NO: 2) and approximately 1 kbp regions upstream and downstream therefrom were amplified by PCR using genomic DNA of *Cupriavidus necator* strain H16 (NC_008313) as template and using the oligonucleotides of Sequence 9 and Sequence 10 indicated below as primers. PCR was carried out for 30 cycles consisting of reacting for 20 seconds at 98°C, 15 seconds at 65°C and 3 minutes at 68°C using KOD Plus. The amplified fragment was purified with a DNA purification kit.
Sequence 9: CCCAAGCTTCGAGATCGACCGCGAGTTGTCG (SEQ ID NO: 12)
   (Underline indicates HindIII restrictase site)
Sequence 10: CTAGTCTAGACAAAGGCCTCGTTCAGTTCGATC (SEQ ID NO: 13) (Underline indicates XbaI restrictase site)

Subsequently, the 5'-terminal was phosphorylated by T4 kinase, and ligated with pUC118, subjected to HincII cleavage and alkaline phosphatase treatment, with Ligation High. A fragment was then amplified containing an approximately 1 kbp region upstream from fadB', a pUC118 vector region and an approximately 1 kbp region downstream from fadB' by inverse PCR using the resulting plasmid as template and using oligonucleotides of the following Sequence 11 and Sequence 12 as primers. PCR was carried out for 30 cycles consisting of 20 seconds at 98°C, 15 seconds at 65°C and 3 minutes at 68°C using KOD Plus. The amplified fragment was purified with a DNA purification kit.
Sequence 11: GCTGGTTCCTTTGGTGTCAAAAGTTGTCTCG (SEQ ID NO: 14)
Sequence 12: TACCGCCACATGGTTCAGCCTGGAGCTGAGAT (SEQ ID NO: 15)

Subsequently, the 5'-terminal was phosphorylated by T4 kinase and self-ligated with Ligation High. The resulting vector was cleaved with HindIII and XbaI to isolate a fragment in which the upstream region and downstream region of fadB' were ligated. fadB' disruption vector pK18msΔfadB' was obtained by ligating this fragment with pK18mobSacB that had been cleaved with HindIII and XbaI.

### Example 6 Production of Homologous Recombinant Strains

The recombinant *Cupriavidus necator* strains described in Example 1 were transformed by conjugal transfer using each of the vectors obtained in Example 2. First, each vector was introduced into Escherichia coli strain S17-1 by the calcium chloride method. Next, this recombinant Escherichia coli was cultured overnight at 37°C in 3.0 ml of LB medium (1% tryptone, 1% sodium chloride, 0.5% yeast extract, pH 7.2). In parallel therewith, the recombinant *Cupriavidus necator* strains were cultured overnight at 30°C in 3.0 ml of NR medium (1% fish meat extract, 1% polypeptone, 0.2% yeast extract). Subsequently, 0.1 ml of recombinant *Cupriavidus necator* strain culture broth was mixed with 0.2 ml of the Escherichia coli culture broth followed by culturing for 6 hours at 30°C. This bacterial mixture was coated onto Simmons Citrate agar medium (Difco Laboratories, Inc.) containing 0.2 mg/ml of kanamycin followed by culturing for 3 days at 30°C. Those bacterial cells in which the recombinant Escherichia coli vector had been transferred to *Cupriavidus necator* and incorporated into a chromosome by homologous recombination demonstrated resistance to kanamycin, while since the recombinant Escherichia coli were unable to grow on the Simmons Citrate agar medium, the colonies that grew on the aforementioned medium consisted of *Cupriavidus necator* transformants (pop-in strains) in which pK18msΔfadB1 had been incorporated into a chromosome from the recombinant Escherichia coli. Moreover, after culturing the pop-in strains overnight at 30°C in NR medium, they were coated onto NR medium containing 10% sucrose followed by culturing for 3 days at 30°C. Levansucrase encoded by sacB in pK18mobsacB causes accumulation of toxic polysaccharides in the cells as a result of using sucrose as substrate. Consequently, only those strains from which the plasmid region had been removed (pop-out strains) were able to survive in the medium containing 10% sucrose. Clones in which fadB1, fadB2 and fadB' had been deleted from chromosomes of these colonies were screened by PCR and were respectively named *Cupriavidus necator* "strain NSDG-ΔfadB1", "strain NSDG-ΔfadB2" and "strain NSDG-ΔfadB'". In addition, double-disrupted strains of fadB1 and fadB2 as well as fadB1 and fadB' were produced using the same method as described above, and were respectively named "strain NSDG-ΔΔfadB1fadB2" and "strain NSDG-ΔΔfadB1fadB'".

### Example 7 Copolymerized Polymer Synthesis by Recombinant Cupriavidus necator Strains

Recombinant *Cupriavidus necator* strains pre-cultured in NR medium (as described above) were inoculated into 100 ml of MB medium (0.9% disodium hydrogen phosphate dodecahydrate, 0.15% potassium dihydrogen phosphate, 0.05% ammonium chloride, 1% trade metal solution) followed by shake-culturing for 72 hours at 30°C in a Sakaguchi flask. 1% soybean oil was used as carbon source. 0.1 mg/ml of kanamycin was added to the medium for recombinant strains retaining a disruption vector. Following completion of culturing, the cells were recovered by centrifugal separation, and after washing with 70% ethanol to remove adhered oil, the cells were washed with distilled water. The resulting cells were freeze-dried followed by measurement of their dry cell mass (DCM).

2 ml of a sulfuric acid-methanol mixture (15:85) and 2 ml of chloroform were added to 10 mg to 30 mg of the dry cells followed by sealing the flask and heating for 140 minutes at 100°C to obtain a methyl ester of the polyester decomposition product within the cells. 1 ml of distilled water was added thereto followed by stirring vigorously. After allowing to stand undisturbed to separate into two layers, the lower organic layer was removed. 0.5 ml of the organic layer was analyzed by capillary gas chromatography. The Model GC-17A manufactured by Shimadzu Corp. was used for the gas chromatograph, and InertCap-1 manufactured by GL Sciences Inc. (column length: 25 m, column inner diameter: 0.25 mm, liquid film thickness: 0.4 µm) was used for the capillary column. Temperature conditions consisted of raising the temperature at a rate of 8°C/min from a starting temperature of 100°C. The results obtained are shown in the following Tables 1 and 2.

[Table 1]

**Table 1 Effects of Disruption of fadB Gene on PHA Production in Recombinant Cupriavidus necator Strains Using Soybean Oil as Carbon Source**

| Strain | Genotype | DCM (g/L) | PHA Content (wt%) | 3HHx (mol%) | RCM (g/L) | PHA (g/L) |
|---|---|---|---|---|---|---|
| NSDG (phaC_{NSGD}-phaAB1) | | 6.79±0.23 | 87.61±2.43 | 1.98±0.07 | 0.93 | 5.96 |
| NSDG-ΔfadB1 | Δfadb1 | 6.59+0.51 | 85.62+3.01 | 2.55±0.09 | 0.94 | 5.65 |
| NSDG-ΔfadB2 | Δfadb2 | 6.36+0.17 | 87.70 | 1.80 | 0.78 | 5.57 |
| NSDG-ΔΔfadB1B2 | ΔfadB1ΔfadB2 | 6.27+0.09 | 83.08+2.64 | 2.52±0.07 | 0.99 | 5.29 |
| NSDG-ΔfadB' | ΔfadB' | 4.86±0.04 | 75.98±4.25 | 3.22±0.13 | 1.07 | 3.75 |
| NSDG-ΔΔfadB1B' | ΔfadB1ΔfadB' | 0.32±0.09 | 7.74±1.13 | 10.77±1.69 | 0.29 | 0.025 |

As is described in the results of Table 1, although the amount of PHA synthesized from soybean oil decreased slightly in the fadB1 disrupted strain, the 3HHx fraction increased from 2.0 mol% to 2.6 mol%. On the other hand, although the 3HHx fraction increased to 3.2 mol% in the fadB' gene-disrupted strain, the amount of PHA produced decreased. Bacterial growth and PHA production decreased in the fadB1-fadB' double-disrupted strain. In addition, since a tendency for 3HHx fraction to increase in the copolymer was observed without a significant reduction in the amount of PHA produced in the fadB1 gene-disrupted strain, a study of PHA production when using soybean oil as carbon source was examined by introducing fadB1 disruption in the same manner as Example 6 for each of the *Cupriavidus necator* strains previously produced by the inventors of the present invention (strain MF02, strain MF03 and strain MF03-J1, see Patent Document 4). As a result, the 3HHx fraction was found to increase by about 1 mol% without exhibiting a significant reduction in the amount of PHA produced in all of the strains (Table 2). Moreover, when the amounts of monomer units incorporated in the polymer were calculated (far right columns in Tables 1 and 2), the incorporated amount of the 3HHx unit was definitely indicated to be increased by disruption of fadB1. This indicates that the change in copolymer composition resulting from this gene disruption is not merely a relative change attributable to a reduction in the 3HB unit.

**[Table 2]**

| PHA Production by Various *Cupriavidus necator* Strains and ΔfadB1 Derivatives from Soybeans | | | | | | |
|---|---|---|---|---|---|---|
| Strain | Genotype | CDW (g/L) | PHA Content (wt%) | 3HHx (mol%) | RCM (g/L) | PHA (g/L) |
| MF02 | phaC_{NSDG}J_{Ac}-phaAB1 | 6.45±0.29 | 87.58±2.80 | 5.24±0.20 | 0.87 | 5.66 |
| MF02-ΔfadB1 | phaC_{NSDG}J_{Ac}-phaAB1ΔfadB1 | 6.46±0.16 | 88.34±1.39 | 6.25±0.04 | 0.75 | 5.70 |
| MF03 | phaC_{NSDG}J_{Ac}-phaB1 | 4.98±0.07 | 85.51+1.30 | 9.39±0.09 | 0.72 | 4.26 |
| MF03-ΔfadB1 | phaC_{NSDG}J_{Ac}-phaB1ΔfadB1 | 4.58+0.25 | 90.00±1.51 | 10.84±0.02 | 0.46 | 4.12 |
| MF03-J1 | phaC_{NSDG}J_{Ac}J4a-phaB1 | 4.72±0.43 | 86.48±2.94 | 10.64±0.23 | 0.63 | 4.09 |
| MF03-J1-ΔfadB1 | phaC_{NSDG}J_{Ac}J4a-phaB1ΔfadB1 | 4.69±0.06 | 82.00±2.15 | 11.74±0.84 | 0.85 | 3.91 |

Although improvement of the 3HHx fractions obtained in the present example was about 1 mol%, the PHA production rates (per dry cell) of the recombinant *Cupriavidus necator* strains used in the present example were extremely high at 80% or more, and it is normally extremely difficult to increase 3HHx fraction while maintaining such high accumulation rates and production rates. In the past, even in the case the 3HHx fraction was able to be increased by various types of genetic manipulation, that increase was actually a relative increase in the 3HHx fraction attributable to a decrease in the fraction of the 3HB unit having four carbon atoms, and was ultimately accompanied by a decrease in the amount of PHA produced. In the present invention, 3HHx fraction is able to be successfully increased while maintaining a high level of PHA production, which is extremely significant from the viewpoint of industrialization.

## Claims

1. A method for producing poly(3-hydroxybutyrate-co-3- hydroxyhexanoate), comprising:
(a) providing a recombinant *Cupriavidus necator* strain imparted with the ability to produce poly(3-hydroxybutyrate-co-3-hydroxyhexanoate),
(b) completely or partially deleting, modifying or disrupting at least one gene encoding 2-enoyl-CoA hydratase or at least one gene encoding 3-hydroxyacyl-CoA dehydrogenase on a chromosome of the strain to suppress expression of the gene, and
(c) growing the strain in medium containing vegetable oil as a carbon source; wherein,
the 3-hydroxyhexanoate composition of poly(3-hydroxybutyrate-co-3-hydroxyhexanoate) is 1 mol% to 20 mol% and the strain internal accumulation rate is 50% by weight to 90% by weight.

2. The method according to claim 1, wherein the recombinant *Cupriavidus necator* strain is a NSDG strain, a NSDGΔA strain, a MF01 strain, a MF03 strain or a MF03-J1 strain.

3. The method according to claim 1 or 2, wherein the gene encoding 2-enoyl-CoA hydratase or the gene encoding 3-hydroxyacyl-CoA dehydrogenase is selected from the group consisting of fadB1 (SEQ ID NO: 1), fadB2 (SEQ ID NO: 2), fadB' (SEQ ID NO: 3), and combinations thereof.

4. The method according to any one of claims 1 to 3, wherein the suppression of gene expression is gene disruption.

5. A method for producing a recombinant *Cupriavidus necator* strain that produces poly(3-hydroxybutyrate-co-3- hydroxyhexanoate) having an improved 3-hydroxyhexanoate fraction, comprising:
(a) providing a recombinant *Cupriavidus necator* strain imparted with the ability to produce poly(3-hydroxybutyrate- co-3-hydroxyhexanoate),
(b) constructing a vector for completely or partially deleting, modifying or disrupting at least one gene encoding 2-enoyl-CoA hydratase or at least one gene encoding 3-hydroxyacyl-CoA dehydrogenase on a chromosome of the strain, and
(c) introducing the vector constructed in (b) into the strain of (a) to suppress function of the gene by disrupting or modifying the gene by homologous recombination.

6. The method according to claim 5, wherein the recombinant *Cupriavidus necator* strain is a NSDG strain, a NSDGΔA strain, a MF01 strain, a MF03 strain or a MF03-J1 strain.

7. The method according to claim 5 or 6, wherein the gene encoding 2-enoyl-CoA hydratase or the gene encoding 3-hydroxyacyl-CoA dehydrogenase is selected from the group consisting of fadB1 (SEQ ID NO: 1), fadB2 (SEQ ID NO: 2), fadB' (SEQ ID NO: 3), and combinations thereof.

8. The method according to any one of claims 5 to 7, wherein the suppression of gene expression is gene disruption.
